(19) **Europäisches Patentamt / European Patent Office / Office européen des brevets**

(11) **EP 4 738 245 A1**

(12) # EUROPEAN PATENT APPLICATION

(43) Date of publication:
**06.05.2026 Bulletin 2026/19**

(21) Application number: **25181046.1**

(22) Date of filing: **05.06.2025**

(51) International Patent Classification (IPC):
**G06T 5/80** *(2024.01)* **G06T 7/11** *(2017.01)*
**G06T 7/64** *(2017.01)* **G06T 7/66** *(2017.01)*

(52) Cooperative Patent Classification (CPC):
**G06T 5/80; G06T 7/11; G06T 7/64; G06T 7/66;**
G06T 2207/10081; G06T 2207/20084;
G06T 2207/30012; G06T 2207/30172

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH LA MA MD TN**

(30) Priority: **31.10.2024 KR 20240152430**
**27.05.2025 KR 20250069295**

(71) Applicant: **Claripi Inc.**
**Seoul 03088 (KR)**

(72) Inventors:
• **Kim, Jong Hyo**
  **02861 Seoul (KR)**
• **Lee, Je Myoung**
  **02423 Seoul (KR)**
• **Kim, Min Beom**
  **03136 Seoul (KR)**

(74) Representative: **Habermann, Hruschka & Schnabel**
**Patentanwälte**
**Montgelasstraße 2**
**81679 München (DE)**

(54) **SYSTEM AND METHOD FOR SAGITTAL PLANE CORRECTION OF MEDICAL IMAGES**

(57) A processing unit configured to reconstruct a shape of a spine structure contained in medical images is provided, segmenting a vertebral body by inputting the medical images to a deep learning model trained in advance; constructing a centerline by connecting central points of segmented vertebral bodies; setting a correction coordinate system based on a sagittal plane by analyzing a curvature of the centerline; generating a sagittal plane cross-section image by transforming the shape of the spine structure based on the correction coordinate system; and outputting the sagittal plane cross-section image as a diagnostic image.

## FIG. 1

1000

100

10

30

communication unit (110)

data storage unit (120)

processing unit (130)

medical imaging device

medical staff

EP 4 738 245 A1

**Description**

BACKGROUND OF THE INVENTION

Field of the Invention

The disclosure relates to a system and method for sagittal plane correction of medical images, and more particularly to a system and method for sagittal plane correction of medical images, in which a spine structure of a scoliosis patient in the medical image is corrected to reconstruct a distorted sagittal plane cross-section into an aligned state.

Description of the Related Art

In general, a curved planar reformation (CPR) technique is used in the diagnosis and evaluation of scoliosis. A computerized photometric radiographic imaging technique uses a computer-based photogrammetric technique to accurately diagnose and evaluate the spinal deformity of a patient. The computerized photometric radiographic imaging technique is useful for the diagnosis and follow-up of scoliosis because it is non-invasive and allows for three-dimensional modeling and accurate angle measurement based on a computer analysis of captured images.

In this computerized photometric radiographic imaging technique, the three-dimensional curvature of a spine is measured by manually specifying the central points of vertebral bodies and generating a curved path. However, in the diagnosis of spinal disorders, it takes a lot of diagnostic time to manually analyze multiple slices, and it is difficult to identify the entire spine at once, thereby limiting the diagnostic efficiency and accuracy.

Documents of Related Art]

[Patent Document]

Korean Patent Publication No. 2024-0042866 (titled "METHOD FOR PROVIDING INFORMATION ON SCOLIOSIS BASED ON ARTIFICIAL INTELLIGENCE," and published on April 2, 2024).

SUMMARY OF THE INVENTION

An aspect of the disclosure is to provide a system and method for sagittal plane correction of medical images, in which a sagittal plane image is automatically reconstructed by correcting the curvature of a spine from the medical images of a scoliosis patient based on a deep learning model, thereby intuitively displaying the entire spine structure.

In accordance with an embodiment of the disclosure, an apparatus for sagittal plane correction of medical images includes: a processing unit configured to reconstruct a shape of a spine structure contained in medical images, wherein the processing unit is configured to: segment a vertebral body by inputting the medical images to a deep learning model trained in advance; construct a centerline by connecting central points of segmented vertebral bodies; set a correction coordinate system based on a sagittal plane by analyzing a curvature of the centerline; generate a sagittal plane cross-section image by transforming the shape of the spine structure based on the correction coordinate system; and output the sagittal plane cross-section image as a diagnostic image.

The deep learning model may incorporate a U-Net or Attention architecture, and perform segmentation for vertebral bodies based on a mask.

The processing unit may derive each central point of the vertebral body by calculating coordinates of the vertebral body, and construct a three-dimensional centerline by connecting the central points of the vertebral bodies.

The processing unit may use at least one of linear interpolation, a cubic Bézier curve, and a cubic spline to construct the three-dimensional centerline.

The processing unit may define a start point and an end point with a straight line in the medical images, and construct the three-dimensional centerline by calculating an intermediate position after identifying a position between the two points.

The processing unit may define a start point and an end point in the medical images, and construct the three-dimensional centerline by identifying a shape of a curve through control points arranged in a curvature direction or tangential direction.

The processing unit may connect the central points of the vertebral bodies in the medical images with a curve, and define a spline curve for each axis of a global coordinate system to construct the three-dimensional centerline.

The processing unit may calculate a tangent vector and a curvature at a preset point in analyzing the curvature of the centerline, and the preset point may include at least one of the central points of the vertebral bodies and interpolated points between the vertebral bodies.

The processing unit may calculate a direction of the centerline based on difference in the central point between the vertebral bodies in calculating the tangent vector.

The processing unit may calculate a degree to which the centerline is bent, based on difference in the central point between the vertebral bodies in calculating the curvature.

The processing unit may use a local coordinate system defined based on the tangent vector and curvature of the centerline in setting the correction coordinate system.

The processing unit may transform the medical images according to the correction coordinate system, and reconstruct the medical images into a sagittal plane cross-section image from which the curvature is removed.

The medical images may include computed tomography images of a thorax or lumbar region.

In accordance with another embodiment of the disclosure, a system for sagittal plane correction of medical images includes: a communication unit configured to acquire medical images; and a processing unit configured to reconstruct a shape of a spine structure contained in the medical images, wherein the processing unit is configured to: segment a vertebral body by inputting the medical images to a deep learning model trained in advance; construct a centerline by connecting central points of segmented vertebral bodies; set a correction coordinate system based on a sagittal plane by analyzing a curvature of the centerline; generate a sagittal plane cross-section image by transforming the shape of the spine structure based on the correction coordinate system; and output the sagittal plane cross-section image as a diagnostic image.

In accordance with still another embodiment of the disclosure, a method of sagittal plane correction of medical images to reconstruct a shape of a spine structure contained in the medical images includes: segmenting a vertebral body by inputting the medical images to a deep learning model trained in advance; constructing a centerline by connecting central points of segmented vertebral bodies; setting a correction coordinate system based on a sagittal plane by analyzing a curvature of the centerline; generating a sagittal plane cross-section image by transforming the shape of the spine structure based on the correction coordinate system; and outputting the sagittal plane cross-section image as a diagnostic image.

Accordingly, the system and method for sagittal plane correction of medical images according to the disclosure have the following effects.

First, the entire spine is reconstructed on an aligned-sagittal plane image, allowing for intuitively confirming the entire structure in the diagnosis of scoliosis.

Second, automatic segmentation and centerline generation are automatically performed based on the deep learning model, thereby automating an analysis process and maximizing a diagnostic efficiency.

Third, reproducibility and diagnostic consistency are improved compared to those of the conventional manual centerline-based computerized photometric radiographic imaging techniques.

Fourth, a quantitative analysis of image data is easily performed, thereby providing efficient and reliable image analysis in various medical fields such as angle measurement, compression fracture diagnosis, and treatment plan establishment.

The foregoing technical effects of the disclosure are not limited to the effects mentioned above, and other technical effects not mentioned will be clearly understood by those skilled in the art from the following description.

BRIEF DESCRIPTION OF THE DRAWINGS

FIG. 1 is a conceptual diagram showing a system for sagittal plane correction of medical images according to an embodiment,

FIG. 2 is a flowchart showing a sagittal plane correction method using a system for sagittal plane correction of medical images according to an embodiment,

FIG. 3 is a conceptual diagram showing a sagittal plane correction method using a sagittal plane correction program installed in a system for sagittal plane correction of medical images according to an embodiment, and

FIG. 4 shows a medical image and a diagnostic image input to a system for sagittal plane correction of medical images according to an embodiment.

DETAILED DESCRIPTION OF THE INVENTION

Below, embodiments of the disclosure will be described in detail with reference to the attached drawings. However, these embodiments are not limited to the embodiments set forth herein, and may be implemented in various forms. Rather, these embodiments are provided so that the disclosure will be thorough and complete, and will fully convey the category of the disclosure to a person having ordinary knowledge in the art. In the drawings, the shapes and the like of elements may be exaggerated for clarity, and like reference numerals in the drawings denote like elements.

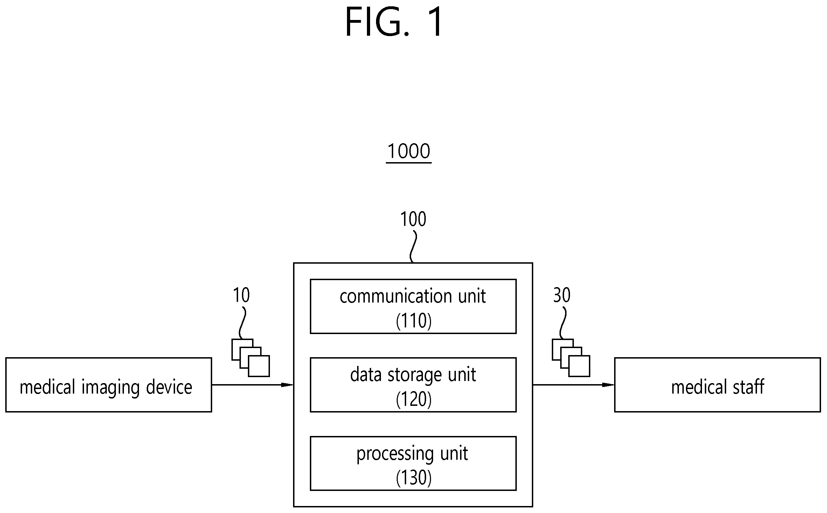

FIG. 1 is a conceptual diagram showing a system for sagittal plane correction of medical images according to an embodiment.

As shown in FIG. 1, a system 1000 for sagittal plane correction of medical images (hereinafter referred to as a correction system) according to the present embodiment includes a sagittal plane correction apparatus 100.

The sagittal plane correction apparatus 100 corrects a spine structure of a patient with a spinal disorder in medical images 10 acquired from a medical imaging device, thereby reconstructing a distorted sagittal plane cross-section into an aligned state. The medical images 10 may be, but not limited to, computed tomography images of a thorax or lumbar region. In addition, the sagittal plane correction apparatus 100 may be configured as an electronic apparatus in which a sagittal plane correction program P can be installed, and may employ various electronic apparatuses including a display device, such as a personal computer (PC), a netbook, a tablet PC, and a smart phone.

Meanwhile, the sagittal plane correction apparatus 100 includes a communication unit 110, a data storage unit 120 and a processing unit 130.

The communication unit 110 receives the medical images 10 from the medical imaging device or a separate storage device. In addition, the data storage unit 120 is configured including a memory, and stores the sagittal plane correction program P. The sagittal plane correction program P may reconstruct a sagittal plane of the medical images 10 and output an enhanced improved diagnostic image 30 to a medical staff. In addition, the processing unit 130 may perform overall control of the sagittal plane correction apparatus 100, and generate and execute a process for the sagittal plane correction based on the sagittal plane correction program P.

Below, a sagittal plane correction method using the correction system 1000 according to an embodiment will be described in detail with reference to the accompanying drawings.

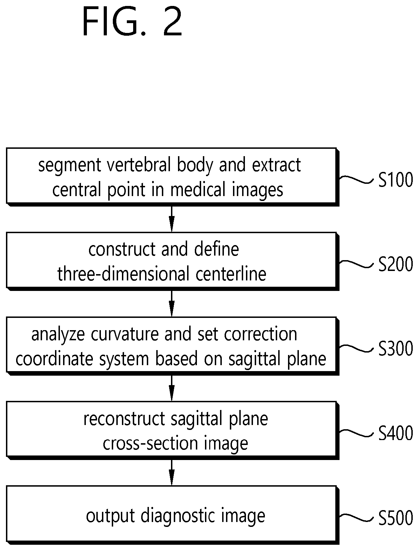

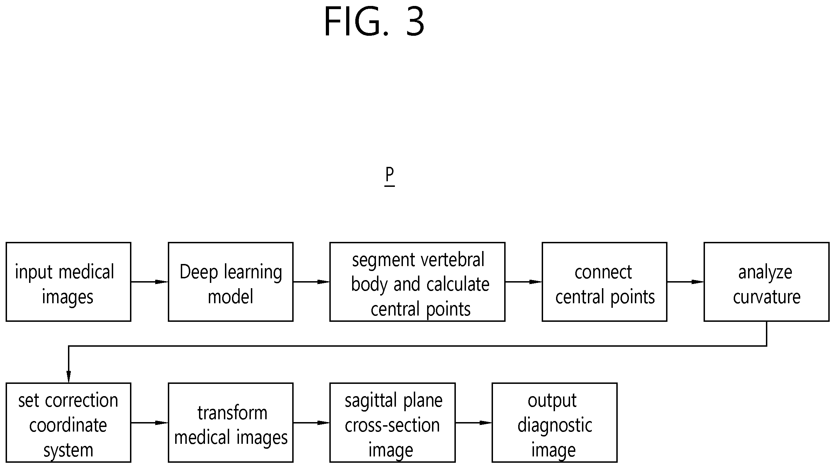

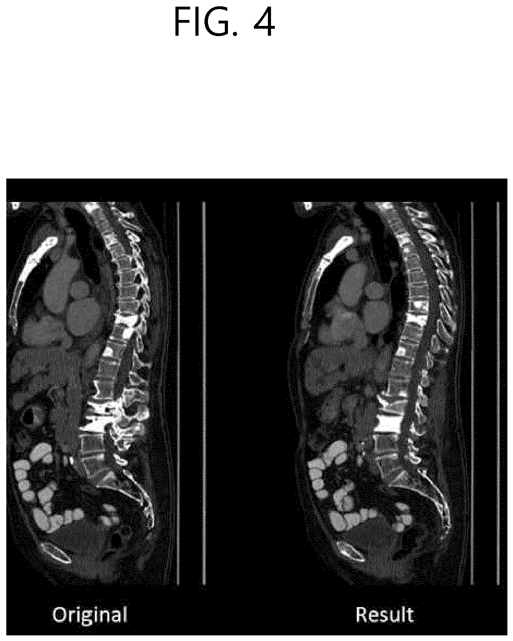

FIG. 2 is a flowchart showing a sagittal plane correction method using a system for sagittal plane correction of medical images according to an embodiment, FIG. 3 is a conceptual diagram showing the sagittal plane correction method using a sagittal plane correction program installed in the system for sagittal plane correction of medical images according to an embodiment, and FIG. 4 shows a medical image and a diagnostic image input to the system for sagittal plane correction of medical images according to an embodiment.

As shown in FIGS. 2 to 4, the sagittal plane correction method using the correction system 1000 according to this embodiment receives the medical images 10 through the communication unit 110. Further, the processing unit 130 may generate and execute the following processor based on the sagittal plane correction program P.

First, the processing unit 130 calls the medical images 10. Then, the processing unit 130 segments a vertebral body contained in the medical images 10 based on the sagittal plane correction program P, and extracts the central point of each vertebral body (S100).

Here, the processing unit 130 may perform automatic segmentation for the vertebral bodies based on a pre-trained deep learning model. The deep learning model incorporating U-Net or Attention architectures may perform the segmentation for the vertebral bodies based on a mask.

For example, the deep learning model with the U-Net architecture may be trained in advance based on medical images for training, such as computed tomography images or magnetic resonance images, containing the vertebral bodies. In this case, the medical images for the training are preprocessed and used as training data for the deep learning model together with a ground-truth vertebral body mask. Thereafter, when the deep learning model with the U-Net architecture has been trained, the processing unit 130 inputs the medical images 10 provided from the communication unit 110 to the deep learning model with the U-Net architecture. Accordingly, the deep learning model with the U-Net architecture may automatically extract the important features of the vertebral bodies, generate the vertebral body mask based on the extracted features, and perform the automatic segmentation of the vertebral bodies.

For another example, the deep learning model with the Attention architecture may be trained in advance based on medical images for training, such as, computed tomography images or magnetic resonance images, containing the vertebral bodies. In this case, the deep learning model with the Attention architecture may be trained to focus on the features of the vertebral bodies by incorporating Attention gates into the model of the U-Net architecture. Then, when the deep learning model with the Attention architecture has been trained, the processing unit 130 inputs the medical images 10 provided from the communication unit 110 to the deep learning model with the Attention architecture. Accordingly, the deep learning model with the Attention architecture may perform the automatic segmentation of the vertebral bodies by evaluating the importance of the features of the vertebral bodies through an Attention gate.

Then, the processing unit 130 mathematically calculates the central points for a plurality of segmented vertebral bodies. For example, the processing unit 130 may derive the central point of each vertebral body by calculating the coordinates of the vertebral body based on the mask generated in the deep learning model. In this case, the central points of each vertebral body may be defined based on the x, y, and z coordinates of each vertebral body. However, this is merely to describe the present embodiment, and the processing unit 130 may use the deep learning model to calculate the central points of the vertebral bodies.

Meanwhile, when the central points of the vertebral bodies are calculated, the processing unit 130 connects the central points of the segmented vertebral bodies based on the sagittal plane correction program P to construct and define a three-dimensional centerline (S200).

In this case, the centerline forms a natural curve of a spine by connecting the central points. To this end, the processing unit 130 may use various interpolation methods to generate a smooth curve.

For example, the processing unit 130 may use linear interpolation, a cubic Bézier curve, a cubic spline, and the like methods to interpolate the centerline.

The linear interpolation refers to a method of connecting the central points of the vertebral bodies with straight lines, in which the processing unit 130 may perform the interpolation with simple straight lines, ignoring the curvature. The linear interpolation is the simplest interpolation method of connecting two points with a straight line, and the connection of the two points with the straight line does not require complex calculations. In addition, the linear interpolation allows for quickly connecting many points because it requires only a start point and an end point without control points or additional data, and is thus very efficient.

Accordingly, the processing unit 130 may perform the linear interpolation based on the following expression 1. That is, the processing unit 130 connects two points with a straight line and calculates an intermediate value on the straight line. In this case, the processing unit 130 may connect a start point ($P_i$) and an end point ($P_{i+1}$) with a straight line, identify a position between the two points with a value ($t$), and then calculate an intermediate position based on the value ($t$).

[Expression 1]

$$P(t) = (1-t)P_i + P_{i+1}$$

$$\text{if } t = \frac{j}{10}:$$

$$x_k = x_1 + \frac{j}{10}(x_2 - x_1), \ y_k = y_1 + \frac{j}{10}(y_2 - y_1), \ z_k = z_1 + \frac{j}{10}(z_2 - z_1)$$

Here, $P_i = (x_1, y_1, z_1)$ represents a start point, and $P_{i+1} = (x_2, y_2, z_2)$ represents an end point, $t$ is a value between 0 and 1, and $j$ may be an integer from 0 to 10. Thus, the processing unit 130 connects the start point and the end point with a straight line and calculates the intermediate value, thereby performing the interpolation.

Meanwhile, the cubic Bézier curve refers to a method of defining a curve with a start point, an end point, and at least two control points, in which the processing unit 130 may adjust the shape of the curve based on the control points. The cubic Bézier curve may naturally adjust the shape of the curve by arranging the control points in the direction of curvature in a region where the curvature is high, thereby representing the natural curvature of the spine.

Accordingly, the processing unit 130 may perform the cubic Bézier curve interpolation based on the following expression 2. That is, the processing unit 130 connects a start point ($P_0$) and an end point ($P_3$) with a curve and calculates an intermediate value on that curve. In this case, the processing unit 130 may define the start point ($P_0$) and the end point ($P_3$), and identify the shape of the curve through the control points ($P_1$, $P_2$) arranged in a curvature direction or a tangent direction. The processing unit may determine a position on the curve with a value $t$, and then calculate an intermediate position of the curve based on the value $t$.

[Expression 2]

$$P(t) = (1-t)^3 P_0 + 3(1-t)^2 t P_1 + 3(1-t)t^2 P_2 + t^3 P_3$$

Here, $P_0$ is a start point that represents the first central point of the vertebral body, $P_3$ is the end point that represents the next central point of the vertebral body, $P_1$ and $P_2$ are control points that represents points to control the shape of the curve, and $t$ is a value between 0 and 1 to determine a position on the curve.

In this case, the processing unit 130 may identify the curved shape of the curve based on the control points ($P_1$, $P_2$), and the control points may be set as in the following expression 3. Here, $\lambda$ is an interpolation coefficient, which may generally be between 0.3 and 0.5.

[Expression 3]

$$P_1 = P_i + \frac{\lambda(p_{i+1} - p_{i-1})}{2}, \quad P_2 = P_{i+1} - \frac{\lambda(p_{i+2} - p_i)}{2},$$

Meanwhile, the cubic spline refers to a method of defining a curve by smoothly connecting multiple points, in which the processing unit 130 may generate a smooth curve by maintaining curvature continuity at each point. The cubic spline allows for generating a smooth and natural curve because the curvature continues successively in sections, and is thus very suitable when the shape of the curve changes continuously like a spine. Because the processing unit 130 automatically adjusts the curvature and direction at each point to generate a consistent curve, there is no need to manually specify separate control points.

Thus, the processing unit 130 may perform the cubic spline interpolation based on the following expression 4. That is, the processing unit 130 connects the central points of the vertebral bodies with a smooth curve and defines a spline curve for each axis (x, y, z) of a global coordinate system. In this case, when three or more central points of the vertebral bodies are given, the processing unit 130 may construct the entire centerline so that curvature continuity can be satisfied for each axis.

[Expression 4]

$$x(t) = S_x(t), \quad y(t) = S_y(t), \quad z(t) = S_z(t),$$

The processing unit 130 applies an independent spline curve to each axis, thereby ensuring that the curves of the axes are smoothly connected without affecting each other. For example, the processing unit 130 may define a spline curve using a cubic polynomial for each axis as in the expression 5.

[Expression 5]

$$x(t) = a_x t^3 + b_x t^2 + c_x t + d_x$$
$$y(t) = a_y t^3 + b_y t^2 + c_y t + d_y$$
$$z(t) = a_z t^3 + b_z t^2 + c_z t + d_z$$

Here, *t* is a value between 0 and 1, which represents a position of a curve, and *a, b, c* and *d* are the coefficients of the spline curve for each axis.

In this case, the processing unit 130 may perform the interpolation to satisfy position continuity, first derivative continuity, second derivative continuity, and an end point boundary condition. The position continuity means that the curve should be continued without breaks at the central point of each vertebral body, and the first derivative continuity means that the slope of the curve should be continuous at the central point of each vertebral body. Further, the second derivative continuity means that the curvature should be smoothly connected at the central point of each vertebral body, and the end point boundary condition means that the slope or curvature of the curve is specified at both ends of the curve, i.e., the first and last vertebral bodies.

Meanwhile, when a three-dimensional centerline is formed, the processing unit 130 analyzes the curvature of the centerline based on the sagittal plane correction program P and sets a correction coordinate system based on the sagittal plane through the curvature (S300).

The curvature of the centerline refers to a value that represents how much the curve is bent, and the processing unit 130 may analyze the curvature of the centerline by calculating a tangent vector ($t_k$) and a curvature ($k$) at each point of the three-dimensional curve. Here, the points where the tangent vector ($t_k$) and curvature ($k$) are calculated may include the central points of the vertebral bodies or the interpolated points between the vertebral bodies.

First, the processing unit 130 may calculate the tangent vector ($t_k$) based on the following expression 6. The tangent vector ($t_k$) represents the direction of a straight line that connect the central points of the vertebral bodies. That is, the processing unit 130 may calculate the tangent vector based on difference in the central point between the vertebral bodies.

[Expression 6]

$$\vec{t_k} = \frac{P_{k+1} - P_{k-1}}{|\overrightarrow{P_{k+1} - P_{k-1}}|}$$

Further, the processing unit 130 may calculate the curvature ($k$) based on the following expression 7. The curvature ($k$) represents how much the central points curve. That is, the processing unit 130 may calculate a degree to which the curve is bent, based on the difference in the position of the central point between the vertebral bodies.

[Expression 7]

$$k_k = \frac{P_{k+1} - 2P_k + P_{k-1}}{|\overrightarrow{P_{k+1} - P_{k-1}}|}$$

In the expressions 6 and 7, $p_k = (x_k, y_k, z_k)$ represents the coordinates of the center of the kth vertebral body, $t_k$ represents a local tangent vector of the central point, and $k$ represents the curvature of the kth centerline.

Meanwhile, when curvature information is acquired, the processing unit 130 sets a correction coordinate system based on the curvature information.

The correction coordinate system refers to a coordinate system where an aligned state is correctly represented according to the centerline of each vertebral body, and may include a local base correction coordinate system defined based on the tangent vector and curvature of the centerline. For instance, the local base correction coordinate system may be defined as a TNB coordinate system, and the TNB coordinate system may construct the correction coordinate system using a tangent vector, a normal vector, and a binormal vector. This correction coordinate system shows a state that the vertebral bodies are aligned with the curve based on the curvature.

Accordingly, the processing unit 130 transforms the medical images 10 based on the sagittal plane correction program P according to the correction coordinate system, and reconstructs the medical images 10 into a sagittal plane cross-section image from which the curvature is removed (S400). That is, the processing unit 130 performs alignment based on the sagittal plane so that the vertebral bodies contained in the medical images 10 can be automatically aligned according to the correction coordinate system. The alignment based on the sagittal plane aligns the vertebral bodies, thereby removing distortion due to the curvature, and making it easy to analyze the shape, alignment, and spacing of the vertebral bodies. In addition, the inclination, misalignment, and deformation of the spine are accurately analyzed, thereby facilitating the diagnosis and treatment of a scoliosis patient.

Meanwhile, when the sagittal plane cross-section image is reconstructed, the processing unit 130 outputs the reconstructed sagittal plane image, i.e., enhanced diagnostic image 30, through the sagittal plane correction program, thereby facilitating each diagnosis of spine disorders such as scoliosis and spondylolisthesis (S500).

Accordingly, the system and method for sagittal plane correction of medical images according to the disclosure have the following effects.

First, the entire spine is reconstructed on an aligned-sagittal plane image, allowing for intuitively confirming the entire structure in the diagnosis of scoliosis.

Second, automatic segmentation and centerline generation are automatically performed based on the deep learning model, thereby automating an analysis process and maximizing a diagnostic efficiency.

Third, reproducibility and diagnostic consistency are improved compared to those of the conventional manual centerline-based computerized photometric radiographic imaging techniques.

Fourth, a quantitative analysis of image data is easily performed, thereby providing efficient and reliable image analysis in various medical fields such as angle measurement, compression fracture diagnosis, and treatment plan establishment.

The embodiments of the disclosure described above and illustrated in the drawings should not be construed as limiting the technical spirit of the disclosure. The scope of the disclosure is limited only by the matters disclosed in the appended claims, and the technical spirit of the disclosure can be modified in various forms by a person having ordinary skill in the art. Accordingly, such modification and change will fall within the scope of the disclosure as long as they are obvious to those skilled in the art.

**Claims**

1. An apparatus for sagittal plane correction of medical images, the apparatus comprising:

   a processing unit configured to reconstruct a shape of a spine structure contained in medical images, wherein the processing unit is configured to:

   segment a vertebral body by inputting the medical images to a deep learning model trained in advance;
   construct a centerline by connecting central points of segmented vertebral bodies;
   set a correction coordinate system based on a sagittal plane by analyzing a curvature of the centerline;
   generate a sagittal plane cross-section image by transforming the shape of the spine structure based on the correction coordinate system; and
   output the sagittal plane cross-section image as a diagnostic image.

2. The apparatus of claim 1, wherein the deep learning model incorporates a U-Net or Attention architecture, and performs segmentation for vertebral bodies based on a mask.

3. The apparatus of claim 2, wherein the processing unit derives each central point of the vertebral body by calculating coordinates of the vertebral body, and constructs a three-dimensional centerline by connecting the central points of the vertebral bodies.

4. The apparatus of claim 3, wherein the processing unit uses at least one of linear interpolation, a cubic Bézier curve, and a cubic spline to construct the three-dimensional centerline.

5. The apparatus of claim 3, wherein the processing unit defines a start point and an end point with a straight line in the medical images, and constructs the three-dimensional centerline by calculating an intermediate position after identifying a position between the two points.

6. The apparatus of claim 3, wherein the processing unit defines a start point and an end point in the medical images, and constructs the three-dimensional centerline by identifying a shape of a curve through control points arranged in a curvature direction or tangential direction.

7. The apparatus of claim 3, wherein the processing unit connects the central points of the vertebral bodies in the medical images with a curve, and defines a spline curve for each axis of a global coordinate system to construct the three-dimensional centerline.

8. The apparatus of claim 1, wherein

   the processing unit calculates a tangent vector and a curvature at a preset point in analyzing the curvature of the centerline, and
   the preset point comprises at least one of the central points of the vertebral bodies and interpolated points between the vertebral bodies.

9. The apparatus of claim 8, wherein the processing unit calculates a direction of the centerline based on difference in the central point between the vertebral bodies in calculating the tangent vector.

10. The apparatus of claim 8, wherein the processing unit calculates a degree to which the centerline is bent, based on difference in the central point between the vertebral bodies in calculating the curvature.

11. The apparatus of claim 8, wherein the processing unit uses a local coordinate system defined based on the tangent vector and curvature of the centerline in setting the correction coordinate system.

12. The apparatus of claim 11, wherein the processing unit transforms the medical images according to the correction coordinate system, and reconstructs the medical images into a sagittal plane cross-section image from which the curvature is removed.

13. The apparatus of claim 1, wherein the medical images comprise computed tomography images of a thorax or lumbar region.

14. A system for sagittal plane correction of medical images, the system comprising:

a communication unit configured to acquire medical images; and
a processing unit configured to reconstruct a shape of a spine structure contained in the medical images, wherein the processing unit is configured to:

segment a vertebral body by inputting the medical images to a deep learning model trained in advance;
construct a centerline by connecting central points of segmented vertebral bodies;
set a correction coordinate system based on a sagittal plane by analyzing a curvature of the centerline;
generate a sagittal plane cross-section image by transforming the shape of the spine structure based on the correction coordinate system; and
output the sagittal plane cross-section image as a diagnostic image.

15. A method of sagittal plane correction of medical images to reconstruct a shape of a spine structure contained in the medical images, the method comprising:

segmenting a vertebral body by inputting the medical images to a deep learning model trained in advance;
constructing a centerline by connecting central points of segmented vertebral bodies;
setting a correction coordinate system based on a sagittal plane by analyzing a curvature of the centerline;
generating a sagittal plane cross-section image by transforming the shape of the spine structure based on the correction coordinate system; and
outputting the sagittal plane cross-section image as a diagnostic image.

# FIG. 1

1000

medical imaging device

10

100

communication unit
(110)

data storage unit
(120)

processing unit
(130)

30

medical staff

# FIG. 2

```
segment vertebral body and extract
central point in medical images          S100

construct and define
three-dimensional centerline             S200

analyze curvature and set correction
coordinate system based on sagittal plane  S300

reconstruct sagittal plane
cross-section image                      S400

output diagnostic image                  S500
```

# FIG. 3

P

| input medical images | → | Deep learning model | → | segment vertebral body and calculate central points | → | connect central points | → | analyze curvature |
|---|---|---|---|---|---|---|---|---|

| set correction coordinate system | → | transform medical images | → | sagittal plane cross-section image | → | output diagnostic image |
|---|---|---|---|---|---|---|

# FIG. 4

Original                                          Result

Europäisches
Patentamt

European
Patent Office

Office européen
des brevets

## EUROPEAN SEARCH REPORT

**Application Number**

EP 25 18 1046

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | CN 116 128 754 A (BEIJING CHANGMUGU MEDICAL TECHNOLOGY CO LTD; ZHANG YILING) 16 May 2023 (2023-05-16) * paragraph [0066] - paragraph [0096] * ----- | 1-15 | INV. G06T5/80 G06T7/11 G06T7/64 G06T7/66 |
| A | CN 112 884 786 A (HANGZHOU JIANPEI TECH CO LTD) 1 June 2021 (2021-06-01) * paragraph [0023] - paragraph [0052] * * figures 8-10 * ----- | 1-15 | |
| A | KRISHNARAJ ARUN ET AL: "Simulating Dual-Energy X-Ray Absorptiometry in CT Using Deep-Learning Segmentation Cascade", JOURNAL OF THE AMERICAN COLLEGE OF RADIOLOGY, ELSEVIER, AMSTERDAM, NL, vol. 16, no. 10, 12 April 2019 (2019-04-12), pages 1473-1479, XP085846821, ISSN: 1546-1440, DOI: 10.1016/J.JACR.2019.02.033 [retrieved on 2019-04-12] * page 1473 - page 1475 * ----- | 1-15 | |

**TECHNICAL FIELDS SEARCHED (IPC)**

G06T

The present search report has been drawn up for all claims

| Place of search | Date of completion of the search | Examiner |
|---|---|---|
| Munich | 31 October 2025 | Maier, Werner |

EPO FORM 1503 03.82 (P04C01)

EP 4 738 245 A1

**ANNEX TO THE EUROPEAN SEARCH REPORT
ON EUROPEAN PATENT APPLICATION NO.**

EP 25 18 1046

This annex lists the patent family members relating to the patent documents cited in the above-mentioned European search report.
The members are as contained in the European Patent Office EDP file on
The European Patent Office is in no way liable for these particulars which are merely given for the purpose of information.

31-10-2025

| Patent document cited in search report | Publication date | Patent family member(s) | Publication date |
|---|---|---|---|
| CN 116128754 A | 16-05-2023 | NONE | |
| CN 112884786 A | 01-06-2021 | NONE | |

EPO FORM P0459

For more details about this annex : see Official Journal of the European Patent Office, No. 12/82

**EP 4 738 245 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 20240042866